Europäisches Patentamt

European Patent Office

Office européen des brevets

(19)

(11) Veröffentlichungsnummer : **0 164 379**
**B1**

(12) **EUROPÄISCHE PATENTSCHRIFT**

(45) Veröffentlichungstag der Patentschrift :
28.09.88

(51) Int. Cl.⁴ : **G 01 N 21/64, G 01 N 31/22**

(21) Anmeldenummer : 85900095.2

(22) Anmeldetag : 01.12.84

(86) Internationale Anmeldenummer :
**PCT/EP 84/00383**

(87) Internationale Veröffentlichungsnummer :
**WO/8502679 (20.06.85 Gazette 85/14)**

(54) **VERFAHREN ZUM NACHWEIS VON BIS-(2-CHLORETHYL)-SULFID ODER BIS-(2-CHLORETHYL)-IMIN.**

(30) Priorität : 10.12.83 DE 3344700

(43) Veröffentlichungstag der Anmeldung :
18.12.85 Patentblatt 85/51

(45) Bekanntmachung des Hinweises auf die Patenterteilung : 28.09.88 Patentblatt 88/39

(84) Benannte Vertragsstaaten :
AT BE CH FR GB LI NL SE

(56) Entgegenhaltungen :
CH-A- 184 663
DE-A- 2 947 459
US-A- 3 960 759
US-A- 4 083 692

(73) Patentinhaber : **BATTELLE DEVELOPMENT CORPO-
RATION**
**505 King Avenue**
**Columbus, OH 43201 (US)**

(72) Erfinder : **KRIEGER, Wolfram**
**Salomon-Heine-Weg 48 a**
**D-2000 Hamburg 20 (DE)**
Erfinder : **ROSSMANN, Klaus**
**Weissacker 111**
**CH-4511 Rumisberg (DE)**
Erfinder : **BOSCHER, Jörg**
**Brunnenweg 1**
**D-2308 Preetz (DE)**
Erfinder : **DIEHL, Wolfgang**
**Stupanusstrasse 3**
**D-6230 Frankfurt am Main 80 (DE)**

(74) Vertreter : **Sartorius, Peter, Dipl.-Ing.**
**Battelle-Institut e.V. Abteilung Patente Am Römerhof
35**
**D-6000 Frankfurt am Main 90 (DE)**

## Beschreibung

Die Erfindung betriff ein Verfahren zum Nachweis von Bis-(2-chlorethyl)-sulfid oder Bis-(2-chlorethyl)-imin durch Zugabe eines chemischen Reagenzes auf die Oberfläche, auf der die nachzuweisende Substanz vermutet wird, und optische Erfassung der entstehenden Strahlung.

Bei dem Bis-(2-chlorethyl)-sulfid bzw. -imin handelt es sich um den Kampfstoff LOST, dessen Detektion auf Oberflächen üblicherweise mit Indikatoren erfolgt, die in Papier oder Pulver eingearbeitet sind. Ebenso wird LOST durch Gaschromatographie nach erfolgter Extraktion der Oberfläche nachgewiesen. Ferner sind Farbreaktionen bekannt, die sich in wässriger oder organischer Lösung vollziehen. Diese Reaktionen werden im Reagenzglas durchgeführt.

Aus der DE-PS-2 947 459 ist eine Vorrichtung zum in-situ-Nachweis von Niederschlägen spezieller seßhafter Kampfstoffe aus der Gruppe der Phosphorsäureester bekannt. Diese Verbindungen zeigen eine Chemielumineszenz unter Einsatz von Indol, die berührungslos mittels eines optischen Detektors nachgewiesen werden kann. Für den Kampfstoff LOST versagt jedoch diese Reaktion.

Der vorliegenden Erfindung liegt nun die Aufgabe zugrunde, eine einfach durchzuführende Nachweismethode für den Kampfstoff LOST zu entwickeln.

Diese Aufgabe ist erfindungsgemäß dadurch gelöst, daß als chemisches Reagenz ein fluoreszenzfähiger Triphenylmethin-Farbstoff verwendet wird, der mit Bis-(2-chlorethyl)-sulfid oder Bis-(2-chlorethyl)-imin ein Addukt bildet, das in einem anderen Spektralbereich als der Farbstoff fluoreszenzfähig ist. Vorzugsweise wird als Farbstoff ein Triphenylmethin-Farbstoff, insbesondere Neutral Rot, Guinea Grün B, Lissamine Grün B oder Brillant Blau R verwendet. Diese Farbstoffe werden aus wässrig/organischer oder organischer Lösung auf die Oberfläche gebracht, auf der der Kampfstoff vermutet wird. Die Menge des Farbstoffs in Lösung beträgt 0,001 bis 0,5 Gew.-%, vorzugsweise 0,01 bis 0,1 Gew.-%.

Erfindungsgemäß können alle Farbstoffe verwendet werden, die ein reaktives Zentrum besitzen, das LOST nucleophil oder elektrophil angreift und zersetzt oder LOST wird adsorptiv unter Konformationsänderung gebunden oder in einer Einschlußverbindung eingebaut. Dadurch verändert sich die Eigenfluoreszenz des Farbstoffs.

Die Anregungswellenlänge sollte vorzugsweise im Bereich zwischen 300 und 400 nm liegen. Die Emissionswellenlänge beträgt vorzugsweise 380 bis 500 nm. Als Lösungsmittel für den Farbstoff sind besonders geeignet Wasser/Alkohol, Wasser/Aceton- oder Wasser/Dimethylformamid-Gemische oder auch Alkohol, Aceton oder Hexan ohne Wasserzugabe.

Die Erfindung wird anhand nachfolgender Beispiele näher erläutert.

Beispiel 1

Neutral Rot wird zu $10^{-3}$ Gew.-% in Ethanol gelöst. Mit 5 µl Sulfid-LOST wird ein Addukt erhalten, das bei Anregung mit Licht der Wellenlänge 330 nm eine Fluoreszenz bei 392 nm mit der relativen Intensität von 1 500 ergibt.

Beispiel 2

Das Verfahren des Beispiels 1 wird wiederholt, wobei jedoch diesmal als Farbstoff Guinea Grün B verwendet wird. Das Addukt zeigt bei Anregung mit Licht der Wellenlänge 320 nm eine Fluoreszenz bei 394 mit der relativen Intensität von 11 700.

Beispiel 3

Lissamine Grün B wird zu $10^{-3}$ Gew.-% in 70 % Wasser — 30 % Ethanol gelöst. Mit 5 µl LOST entsteht ein Addukt, das bei Anregung mit Licht der Wellenlänge 340 nm eine Fluoreszenz bei 470 nm mit der relativen Intensität von 810 zeigt.

Beispiel 4

Brillant Blau R wird zu $2 \times 10^{-2}$ Gew.-% in 70 % Wasser — 30 % Ethanol gelöst. Das Addukt zeigt bei Anregung mit Licht der Wellenlänge 370 nm eine Fluoreszenz bei 455 nm mit der relativen Intensität von 28 100.

## Patentansprüche

1. Verfahren zum Nachweis von Bis-(2-chlorethyl)-sulfid oder Bis-(2-chlorethyl)-imin durch Zugabe eines chemischen Reagenzes auf eine Oberfläche, auf der die nachzuweisende Substanz vermutet wird, und optische Erfassung der entstehenden Strahlung, dadurch gekennzeichnet, daß als chemisches Reagenz ein fluoroeszenzfähiger Triphenylmethin-Farbstoff verwendet wird, der mit Bis-(2-chlorethyl)-sulfid oder Bis-(2-chlorethyl)-imin ein Addukt bildet, das in einem anderen Spektralbereich als Farbstoff fluoreszenzfähig ist.

2. Verfahren nach Anspruch 1, dadurch gekennzeichnet, daß als Farbstoff Neutral Rot, Guinea Grün B, Lissamine Grün B oder Brillant Blau R verwendet wird.

3. Verfahren nach einem der Ansprüche 1 oder 2, dadurch gekennzeichnet, daß der Farbstoff in wässrig/organischer oder organischer Lösung zugegeben wird, die 0,5 bis 0,001 Gew.-%, vorzugsweise 0,1 bis 0,01 Gew.-% Farbstoff enthält.

**Claims**

1. Method of detecting bis-(2-chloroethyl)-sulphide or bis-(2-chloroethyl)-imine by applying a chemical reagent to a surface on which the substance to be detected is assumed to be present, and of optically determining the radiation generated, comprising : using a triphenyl methine dyestuff being capable of fluorescing and forming an adduct with bis-(2-chloroethyl)-sulphide or bis-(2-chloroethyl)-imine, said adduct being capable of fluorescing in a spectral range different from that of said dyestuff.

2. Method as claimed in Claim 1 wherein neutral red, guinea grenn B., lissamine green B or brilliant blue R is used as dyestuff.

3. Method as claimed in Claim 1 or Claim 2 wherein the dyestuff is applied in aqueous/organic or organic solution containing the dyestuff in amounts between 0,5 and 0,001 weight percent, preferably between 0,1 and 0,01 weight percent.

**Revendications**

1. Procédé pour mettre en évidence le sulfure de bis(2-chloroéthyle) ou la bis(2-chloroéthyle)-imine par addition d'un réactif chimique à une surface, sur laquelle la substance à détecter est supposée être présente, ainsi qu'un enregistrement optique du rayonnement résultant, caractérisé en ce que comme réactif chimique on utilise un colorant du type triphénylméthine capable de fluorescence qui forme un adducteur avec le sulfure de bis(2-chloroéthyle) ou la bis(2-chloroéthyl-imine), susceptible d'être fluorescent dans une zone du spectre que celle du colorant.

2. Procédé selon la revendication 1, caractérisé en ce que l'on utilise comme colorant le Rouge Neutre, le Vert de Guinée, le Vert Lissamine B ou le Bleu Brillant R.

3. Procédé selon l'une des revendications 1 ou 2, caractérisé en ce que le colorant est ajouté en solution aqueuse/organique ou organique, contenant de 0,5 à 0,001 % en poids, de préférence de 0,1 à 0,01 % en poids de colorant.